# EUROPEAN PATENT SPECIFICATION

(11) **EP 2 517 693 B1**
(45) Date of publication and mention of the grant of the patent: **23.05.2018**
(21) Application number: 11163702.1
(22) Date of filing: 26.04.2011
(51) Int. Cl.: A61K 8/45, A61Q 9/02, A61Q 19/00, B26B 21/44

(54) **Hair removal device comprising a modifying surfactant**
Haarentfernungsvorrichtung mit einem modifizierten Tensid
Dispositif d'épilation comprenant un agent tensioactif à modification

(43) Date of publication of application: 31.10.2012
(73) Proprietor: The Gillette Company LLC, Boston, MA 02127 (US)
(72) Inventor: Stephens, Alison, Fiona, Maidenhead, Berkshire SL6 9LA (GB); Baxter, Elaine, Alice, Marie, St Margarets, Middlesex TW1 2QX (GB); Brooks, Alan, Woking, Surrey GU21 8UQ (GB)
(74) Representative: Kohol, Sonia

(56) References cited:
- WO-A1-2007/056509
- WO-A2-2009/114420
- WO-A2-2011/022321
- GB-A- 2 423 494

## Description

### FIELD OF THE INVENTION

The present invention concerns the provision of a hair removal device comprising an erodible moisturizer.

### BACKGROUND OF THE INVENTION

Hair removal devices incorporating a chemical composition are known and shall be referred to herein as devices comprising an "onboard" composition. Reference can be made to WO 07/056509 which teaches the inclusion of an onboard soap composition in a wet shaving razor. It is also known to provide a wet shaving razor incorporating an onboard skin-engaging composition comprising large quantities of hydrophilic polymers, such as polyethylene oxide, to lubricate the skin. Reference is made, by way of example, to WO 97/02116 and WO 97/02117.

The patent applications referred to above relate to the provision of various advantages, such as additional lathering and soap-related benefits, or improved lubrication in the case of polyethylene oxide. It would alternatively or additionally be advantageous to be able to provide a skin moisturizing benefit via an onboard chemistry, especially to male users who may be less motivated to use skin moisturizers than females. The provision of a moisturizing benefit from an onboard chemistry may, however, have a number of difficulties associated with it.

Skin moisturization may be achieved in several different ways, but one important formulational route to achieving skin moisturization is to include materials which bind water, such as polyols. However, polyols derive their water-binding abilities in part from their significant hydrophilicity, which may render them unsuited for use in any context involving water, such as is the case during wet shaving - they may be washed away during the initial stages of a shave. An alternative approach might be to use occlusive, hydrophobic materials which cover the skin and therefore act to retain water already present in the skin. These materials are emollients which are less likely to be washed away during use in a highly aqueous environment. WO 06/108522, discloses the use of small amounts of hydrophobic emollients in an onboard chemistry. However, formulations comprising significant proportions of hydrophobic emollients generally have mechanical and/or rheological properties which render them unsuitable for use as an onboard chemistry. For example, many hydrophobic emollients are liquids or low viscosity gels which flow easily and/or whose integrity may become substantially impaired by contact with hair, especially stiff male beard hair. One possible result is that the emollient formulation may be mostly or entirely delivered at the start of the first use, over-delivering for that first use and leaving little or nothing for future uses of the hair removal device. A further problem associated with compositions comprising significant proportions of hydrophobic emollients is that they may increase drag across the skin, due to their affinity with the hydrophobic skin surface. Users report that increased drag tends to increase discomfort during shaving.

US 2009/0223057 discloses shaving aid strip compositions comprising polyoxyethylene for similar reasons to the above-discussed prior art. In order to improve the longevity of the shaving aid, the polyoxyethylene is mixed with amphipathic, but generally hydrophobic materials, such as fatty alcohols. Fatty alcohols *per se* may create a sticky, non-lubricious system on skin which increases razor drag leading to the associated negative experiences discussed above. G 2423494 discloses a hair removal device comprising an erodable solid moisturizer comprising a surfactant (SLES (2EO)) and a hydrophobic phase These compositions are taught to be mild to the skin, moisturization is also mentioned.

### SUMMARY OF THE INVENTION

According to a first aspect of the invention, a hair removal device is provided comprising an erodable solid moisturizer, the erodable solid moisturizer comprising:
a. A saturated modifying surfactant comprising:
   i. A carbon chain with 16 to 20 carbon atoms;
   ii. From 1 to 19 ethylene oxide groups;
b. At least 50% hydrophobic phase by weight of the erodable solid moisturizer. According to a second aspect of the invention, the use of a hair removal device according to the first aspect to remove hair and moisturize the skin is provided.

### DETAILED DESCRIPTION OF THE INVENTION

As used herein, the term "solid" when used in relation to the erodable solid moisturizer refers to compositions which are solid at 25°C.
As used herein, the term "water-insoluble" when used in relation to the structuring polymer, means "very slightly soluble", according to the United States' Pharmacopeia (USP) definition in 31/NF 26 Vol. 2 General Notices, Page Xvii., or less than "very slightly soluble", which, using the USP definition, means that more than 1000 parts of solvent (water, in this case) are needed to dissolve 1 part of solute (the structuring polymer, in this case) at Standard Temperature and Pressure.

As used herein, the term "soluble in" when describing the ability of the water-insoluble structuring polymer to dissolve in the hydrophobic phase means "soluble", according to the United States' Pharmacopeia definition in 31/NF 26 Vol. 2 General Notices, Page Xvii., or less than "soluble", which, using the USP definition, means that less than 30 parts of solvent (the hydrophobic phase, in this case) are needed to dissolve 1 part of solute (the structuring polymer, in this case) at the melting point of the water-insoluble structuring polymer.

The hair removal device according to the invention may be any hair removal device, such as, but not limited to, a razor, an epilator or a hair removal device comprising a light source to degrade or destroy the hair and/or hair root. If the hair removal device comprises a light source, then the light source may generate coherent (laser) light or incoherent light and may be adapted to generate light continuously or in a discontinuous (pulsed) fashion.

Preferably, the hair removal device is a razor. In the case of a razor, then the solid moisturizer may advantageously be provided on the razor cartridge, is preferably configured as a strip located before and/or after the blade(s) in the direction of cutting and may even be configured as a ring entirely surrounding the blades. WO 97/02116, referred to above, illustrates locations in which such a strip may be placed.

The present applicants have established that a hair removal device comprising an erodable solid moisturizer rich in hydrophobic moisturizing materials, but also comprising the defined modifying surfactant, results in a significant reduction in drag when compared with compositions which do not comprise the defined modifying surfactant. Reference is made to the coefficient of friction data provided with the working examples: the composition comprising no modifying surfactant (Comparative Example) has a significantly higher coefficient of friction than the two compositions which do.

The modifying surfactant according to the invention has a carbon chain with 16 to 20 carbon atoms and has from 1 to 19 ethylene oxide groups. Advantageously, the erodable solid moisturizer comprises from 1% to 12%, preferably 3% to 11%, more preferably from 4% to 10% of modifying surfactant by weight of the erodable moisturizer. Highly advantageously, the modifying surfactant comprises from 1 to 10, even more advantageously two ethylene oxide groups. Beneficially, the modifying surfactant is saturated, meaning that it comprises only single bonds between the carbon atoms of the carbon chain.

Advantageously, the erodable solid moisturizer comprises no surfactant apart from the saturated modifying surfactant.
Preferably, the erodable solid moisturizer comprised within a hair removal device according to the invention has a Chatillon Hardness at 25°C of 0.50 - 3.25kg, preferably 0.75 - 3.00kg, more preferably 1.00 - 2.50kg, measured according to the protocol provided hereinbelow. Within these ranges, beneficial rates of wear may be achieved in use.
Advantageously, the erodable solid moisturizer additionally comprises a water-insoluble structuring polymer. The presence of such a polymer may bestow harness characteristics within the above-defined ranges to the erodible solid moisturizer, thereby facilitating beneficial rates of wear in use.
The water-insoluble structuring polymer, if present, should not be included as part of the "hydrophobic phase" for the purpose of calculating the percentage of the "hydrophobic phase" by weight of the erodible solid moisturizer.
The water-insoluble structuring polymer comprised within the erodable solid moisturizer may be any water-insoluble structuring polymer which bestows appropriate wear properties to the erodable solid moisturizer and is preferably a water-insoluble structuring polymer which may bestow a Chatillon Hardness in the above-defined ranges to the erodable solid moisturizer. The structuring polymer is water-insoluble to assist miscibility with or solubility in the hydrophobic phase (at the melting point of the water-insoluble structuring polymer), which in turn may ensure a homogenous distribution water-insoluble structuring polymer throughout the hydrophobic phase and thus more even wear properties. In addition, the water insoluble nature of the polymer may improve the durability of the polymer (and therefore also the durability of the erodable solid moisturizer) versus more hydrophilic polymers which may solubilise and wash away during hair removal processes that employ water, such as wet shaving.

According to the invention, the erodable solid moisturizer comprises from 2% to 50%, preferably from 3% to 40%, more preferably 4% to 12% of water-insoluble structuring polymer by weight of the erodable solid moisturizer.

Advantageously, the water-insoluble structuring polymer comprises a block copolymer. More advantageously, the block copolymer comprises a di-block copolymer, a tri-block copolymer, a multi-block copolymer, a radial block copolymer, a random block copolymer, or a mixture of these polymers. More advantageously still, the block copolymer comprises a tri-block copolymer.

In the case in which the block copolymer comprises a tri-block copolymer, then the tri-block copolymer preferably comprises a linear ABA tri-block polymer. Without wishing to be bound by theory, applicants believe that the A blocks aggregate creating domains, within which the hydrophobic phase may accumulate, connected together by the B-blocks. This structure may provide an appropriate hardness to bestow the requisite wear properties to the erodable solid moisturizer, while also being flexible enough to be processed and not to crack or break during processing and/or use.

Advantageously, the linear ABA block copolymer comprises styrene-butadiene-styrene (SBS) block copolymer, styrene-isoprene-styrene (SIS) block copolymer, styrene-ethylenebutylene-styrene (S-EB-S) block copolymer, or mixtures thereof. More advantageously, the linear ABA block copolymer preferably comprises styrene-ethylenebutylene-styrene (S-EB-S) block copolymer. More advantageously still, the weight ratio of styrene to butadiene in the S-EB-S is in the range 20:80 to 40:60 and preferably around 30:70.

Particularly useful commercially available ABA block copolymers include Versagel™ materials available from Penreco and the Kraton™ G series, especially G-6150, G-1651, G-1652 and 1654.

As discussed above, the structuring polymer comprised within the erodable, solid moisturizer may comprise a random block copolymer. An example of a suitable random block copolymer is ethylene vinyl acetate (EVA) which is a copolymer of ethylene and vinyl acetate. Advantageously, the amount of ethylene comprised within the EVA polymer is from 65-90%, preferably from 70-85% by weight of the EVA to give beneficial wear properties. A commercially available range of EVA is called Elvax™, which is commercialised by DuPont.

According to the invention, the erodable solid moisturizer comprises at least 50% hydrophobic phase by weight of the erodable solid moisturizer. Preferably, the erodable solid moisturizer comprises from 60% to 95% and more preferably from 70% to 90% hydrophobic phase by weight of the erodable solid moisturising composition.

The hydrophobic phase may comprise emollient which is liquid, semi-solid and/or solid at room temperature, which emollient may comprise one or more hydrocarbons, fatty acids, fatty alcohols, esters, triglycerides, fats, butters, waxes, lipophilic skin active agents or mixtures thereof.

Advantageously, if solids or semi-solids are present, then the hydrophobic phase comprises less than 20% and preferably less than 5% by weight of the solid moisturizer of materials, and more preferably no materials at all, having a melting point of more than 100°C. This is because excessive quantities of such materials may render the composition inflexible and therefore liable to crack during manufacture and/or use.

Liquid, semi-solid, or solid hydrocarbon emollients which may be comprised within the erodable, solid moisturizer include straight chain, branched chain, saturated and unsaturated hydrocarbons and mixtures thereof and they may comprise natural or synthetic hydrocarbon emollients and mixtures thereof. Preferred natural hydrocarbon emollients include petrolatum, mineral oil and mixtures thereof. Preferred synthetic hydrocarbon emollients include branched chain hydrocarbons, such as isohexadecane (such as Arlamol HD™ from Croda) and Polydecene (such as Puresyn 2™ from Exxon Mobil).

Liquid, semi-solid, or solid fatty alcohol or fatty acid emollients which may be comprised within the erodable, solid moisturizer include saturated and unsaturated higher alcohols, especially C₁₂-C₃₀ fatty alcohols and fatty acids, especially lauric, myristic, palmitic, stearic , arachidic or behenic.

Liquid, semi-solid, or solid ester emollients which may be comprised within the erodable, solid moisturizer include esters of a C₁₂ - C₃₀ alcohol and mixtures thereof, especially isopropyl myristate, isopropyl isostearate and mixtures thereof.

Liquid, semi-solid, or solid triglyceride emollients which may be comprised within the erodable, solid moisturizer include synthetic or natural triglycerides, especially natural triglycerides derived from sunflower, avocado, olive, castor, coconut, cocoa and mixtures thereof. More preferred are coconut-derived triglycerides, such as the commercially available materials Myritol™ 312 and 318 (Cognis), Estasan™ (Croda) and Miglyol™ (Sasol).

Liquid, semi-solid, or solid fat and butter emollients which may be comprised within the erodable, solid moisturizer include coconut butter, shea butter and mixtures thereof.

Liquid, semi-solid, or solid wax emollients which may be comprised within the inventive composition include paraffin wax, microcrystalline wax, candellila, ozokerite and mixtures thereof. Preferably the emollient comprises paraffin wax. Advantageously, hydrophobic phase comprises some wax because waxes may bestow further improved hardness and erodability to the solid moisturising composition, although, as discussed above, the presence of too much wax may render the composition inflexible and therefore liable to crack during manufacture and/or use. Preferably, the solid moisturising composition comprises from 2% to 20% and more preferably from 3% to 15% wax by weight of the solid moisturising composition. The presence of the defined amount of wax may bestow harness characteristics within the above-defined ranges to the erodible solid moisturizer, thereby facilitating beneficial rates of wear in use.

Liquid, semi-solid, or solid lipophilic skin active agent emollients which may be comprised within the inventive composition include oil soluble vitamins, such as vitamin E derivatives, including vitamin E acetate and tocopherol nicotinate; oil-soluble vitamin A derivatives, such as retinyl palmitate; lanolin; ceramides; sterols and sterol esters; salicylic acid; camphor; eucalyptol; essential oils and mixtures thereof.

The erodable, solid moisturizer may comprise one or more additional components which bestow a suitable melt viscosity to the composition, such as oil phase gellants, to facilitate improved processing, provided that the additional component(s) do not significantly reduce the hardness or erodability of the erodable, solid moisturizer. An example of such a component is trihydroxystearin, which is commercially available as Thixcin R™ (manufactured by Elementis Specialities).

Advantageously, the erodable solid moisturizer comprises no polyalkylene oxide.

A erodable, solid moisturizer comprised within the hair removal device of the invention may be manufactured by heating the elements of the hydrophobic phase to a suitable temperature to melt them, typically approximately 130°C, after which, if present, the water-insoluble structuring polymer is added and mixed well until the water-insoluble structuring polymer has dissolved. The mixture is then cooled, typically to approximately 90°C, after which any additional ingredients may be added. In a final step, the mixture is poured into suitable containers or moulds and allowed to cool to room temperature.

Once the mixture has set, it may be affixed to a hair removal device in any appropriate fashion. One such approach would involve mechanically fitting the composition onto the hair removal device. Alternatively, the composition may be directly or indirectly adhered to the hair removal device by means of an adhesive composition. One method of indirect adherence involves casting the solid moisturizer onto a sheet of an appropriate substrate, such as an acetate sheet, which sheet is then adhered to the hair removal device, for example mechanically or via an adhesive.

### Coefficient of Friction Method

Coefficient of friction was measured in a controlled environment in which all friction influencing variables (stroke speed, ramp speed, normal load, stroke length and angle) were constant. The method measured the force required to drag the solid erodible moisturizer over a polyurethane skin mimic having a contact angle of 91° and a surface roughness equivalent to that of skin. The erodible solid moisturizer was cast into 1 mm thick sheets on an acetate background and glued to a razor cartridge-sized piece of plastic.

A fresh piece of a polyurethane skin mimic was used for each experiment; these substrates were warmed to 30°C prior to each experiment. 1ml of room-temperature water was placed on the polyurethane skin mimic and spread evenly across the stroke path. The solid erodible moisturizer was dipped into 21°C water for 20s prior to each experiment. 10 consecutive overlapping strokes were executed with no rinse step. Strokes were taken using the following parameters and drag was measured automatically:

| | |
|---|---|
| Load | 230 g |
| Stroke Speed | Constant at 50 mm/s |
| Stroke Length | 100 mm |

Friction was calculated as drag/load.

### Chatillon Hardness test

Equipment: Chatillon TCD 200 equipped with a digital force gauge

### Sample preparation

1. Fully melt and cast lipid into 60ml weigh boat (70mm X 70mm X 24mm)
2. Store lipid at 25°C overnight to equibrilate
3. Carefully remove lipid from weigh boat prior to hardness testing

### Machine Preparation

A)
   1. Prepare Chatillon TCD 200 and digital force gauge according to manufacturers instructions.
   2. Set the ramp speed to 47 mm / min
B) Measuring the hardness value at 25°C:
   1. The pointed geometry should be attached to the shaft of ramp for this test method.
   2. Place the lipid sample as prepared above and on its side onto the metal base plate directly below the centre of the shaft of the ramp. The mid-point of the lipid should be in line with the centre of the shaft of the ramp.
   3. With the lipid in place below the flat plate the speed set at 47 mm/min and the digital force gauge set at "C Peak" as above, depress the "Down" button on the Chatillon TCD200.
   4. Stop the Chatillon TCD200 just as the probe touches the surface of the lipid and set the distance counter to zero.
   5. Reset the force gauge so that it reads zero
   6. Depress the "Down" button on the Chatillon TCD200 until the distance counter reads 13mm, record C Peak reading.

### Examples

The following examples disclose solid moisturizers, which were incorporated into razors as a strip disposed after the blade in the cutting direction. In use, they were observed to remove hair and deposit the solid moisturizer onto the skin from which hair had been removed.

| | **Example 1** | **Example 2** | **Comparative Example** |
|---|---|---|---|
| Petrolatum | 40.25 | 40.75 | 44.00 |
| Mineral oil | 40.25 | 40.75 | 44.00 |
| Kraton G1650E | 5.00 | 5.00 | 5.00 |
| Thixin R | 2.00 | 2.00 | 2.00 |
| Paraffin Wax SP206 | 7.50 | 6.50 | 5.00 |
| Steareth-2 | 5.00 | | |
| Ceteth-2 | | 5.00 | |
| **Total (100%)** | **100.00** | **100.00** | **100.00** |
| Coefficient of friction | 0.324 | 0.300 | 0.481 |

These compositions were manufactured as follows: the mineral oil was heated using a hot plate and metallic beaker and the petrolatum was added with gentle mixing when the mixture reached a temperature of 50°C. After this, the paraffin wax then the surfactant were added with gentle mixing. In the cases in which Kraton polymer was added, this was done between 120°C and 130°C with gentle mixing for a minimum 30 minutes until complete dissolution had occurred. The mixture was then cooled to 90°C before pouring and molding.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A hair removal device comprising an erodible solid moisturizer, the erodible solid moisturizer comprising :
a. A saturated modifying surfactant comprising :
i. A carbon chain with 16 to 20 carbon atoms;
ii. From 1 to 19 ethylene oxide groups;
b. At least 50% hydrophobic phase by weight of the erodible solid moisturizer.

2. The hair removal device of claim 1, wherein the erodible solid moisturizer comprises from 1% to 12% preferably 3% to 11%, more preferably from 4% to 10% of modifying surfactant by weight of the erodible moisturizer.

3. The hair removal device of any preceding claim, wherein the modifying surfactant comprises two ethylene oxide groups.

4. The hair removal device of any preceding claim, wherein the erodible solid moisturizer has a Chatillon Hardness, measured according to the "Chatillon Hardness Test" described herein of 0.50kg to 3.50kg, preferably 0.75kg to 3.00kg, more preferably 1.00kg to 2.50kg.

5. The hair removal device of any preceding claim, wherein the hydrophobic phase comprises wax and preferably the erodible moisturizer comprises from 2% to 20% and more preferably from 3% to 15% wax by weight of the erodible solid moisturizer.

6. The hair removal device of any preceding claim, wherein the erodible solid moisturizer comprises a water-insoluble structuring polymer.

7. The hair removal device of claim 6, wherein the water-insoluble structuring polymer is soluble in or miscible with the hydrophobic phase at the melting point of the water-insoluble structuring polymer.

8. The hair removal device of claim 6 or 7, wherein the erodible solid moisturizer comprises from 2% to 50%, preferably from 3% to 40%, more preferably 4% to 12% water-insoluble structuring polymer by weight of the erodible solid moisturizer.

9. The hair removal device of any one of claims 7 to 8, wherein the water-insoluble structuring polymer comprises a block copolymer.

10. The hair removal device of any preceding claim, wherein the erodible solid moisturizer comprises from 60% to 95%, preferably from 70% to 90% hydrophobic phase by weight of the erodable solid moisturizer.

11. The hair removal device of any preceding claim, configured as a razor comprising one or more cutting blades.

12. The razor according to claim 11, wherein the erodable solid moisturizer is configured as a strip disposed on one or both sides of the cutting blade(s).

13. Use of a hair removal device according to any preceding claim to remove hair and moisturize the skin.

## Patentansprüche

1. Haarentfernungsvorrichtung, die eine erodierbare, feste Feuchtigkeitscreme umfasst, wobei die erodierbare, feste Feuchtigkeitscreme Folgendes umfasst:
a. ein gesättigtes, modifizierendes Tensid, das Folgendes umfasst:
i. eine Kohlenstoffkette mit 16 bis 20 Kohlenstoffatomen;
ii. von 1 bis 19 Ethylenoxidgruppen;
b. zu mindestens 50 Gew.-% hydrophobe Phase, bezogen auf das Gewicht der erodierbaren, festen Feuchtigkeitscreme.

2. Haarentfernungsvorrichtung nach Anspruch 1, wobei die erodierbare, feste Feuchtigkeitscreme zu 1 Gew.-% bis 12 Gew.-%, vorzugsweise 3 Gew.-% bis 11 Gew.-%, mehr bevorzugt zu 4 Gew.-% bis 10 Gew.-% modifizierendes Tensid, bezogen auf das Gewicht der erodierbaren Feuchtigkeitscreme umfasst.

3. Haarentfernungsvorrichtung nach einem der vorstehenden Ansprüche, wobei das modifizierende Tensid zwei Ethylenoxidgruppen umfasst.

4. Haarentfernungsvorrichtung nach einem der vorstehenden Ansprüche, wobei die erodierbare, feste Feuchtigkeitscreme eine Chatillon-Härte, gemessen nach der hier beschriebenen "Chatillon-Härteprüfung", von 0,50 kg bis 3,50 kg, vorzugsweise 0,75 kg bis 3,00 kg, mehr bevorzugt 1,00 kg bis 2,50 kg aufweist.

5. Haarentfernungseinrichtung nach einem der vorstehenden Ansprüche, wobei die hydrophobe Phase Wachs umfasst und die erodierbare Feuchtigkeitscreme vorzugsweise zu 2 Gew.-% bis 20 Gew.-%, und mehr bevorzugt zu 3 Gew.-% bis 15 Gew.-%, Wachs, bezogen auf das Gewicht der erodierbaren, festen Feuchtigkeitscreme, umfasst.

6. Haarentfernungsvorrichtung nach einem der vorstehenden Ansprüche, wobei die erodierbare, feste Feuchtigkeitscreme ein wasserunlösliches, strukturierendes Polymer umfasst.

7. Haarentfernungsvorrichtung nach Anspruch 6, wobei das wasserunlösliche, strukturierende Polymer am Schmelzpunkt des wasserunlöslichen, strukturierenden Polymers in der hydrophoben Phase löslich oder damit mischbar ist.

8. Haarentfernungsvorrichtung nach Anspruch 6 oder 7, wobei die erodierbare, feste Feuchtigkeitscreme zu 2 Gew.-% bis 50 Gew.-%, vorzugsweise 3 Gew.-% bis 40 Gew.-%, mehr bevorzugt zu 4 Gew.-% bis 12 Gew.-% wasserunlösliches, modifizierendes Tensid, bezogen auf das Gewicht der erodierbaren, festen Feuchtigkeitscreme, umfasst.

9. Haarentfernungsvorrichtung nach einem der Ansprüche 7 bis 8, wobei das wasserunlösliche, strukturierende Polymer ein Blockcopolymer.

10. Haarentfernungseinrichtung nach einem der vorstehenden Ansprüche, wobei die erodierbare, feste Feuchtigkeitscreme zu 60 Gew.-% bis 95 Gew.-%, vorzugsweise zu 70 Gew.-% bis 90 Gew.-% hydrophobe Phase, bezogen auf das Gewicht der erodierbaren, festen Feuchtigkeitscreme, umfasst.

11. Haarentfernungsvorrichtung nach einem der vorstehenden Ansprüche, die als Rasierer ausgeführt ist, der eine oder mehrere Schneidmesser umfasst.

12. Rasierer nach Anspruch 11, wobei die erodierbare, feste Feuchtigkeitscreme als ein Streifen ausgeführt ist, der auf einer Seite oder auf beiden Seiten des einen oder der mehreren Schneidmessers angeordnet ist.

13. Verwendung einer Haarentfernungsvorrichtung nach einem der vorstehenden Ansprüche zum Entfernen von Haaren und um der Haut Feuchtigkeit zu spenden.

## Revendications

1. Dispositif d'épilation comprenant un agent hydratant solide érodable, l'agent hydratant solide érodable comprenant :
a. un agent tensioactif modifiant saturé comprenant :
i. une chaîne carbonée avec 16 à 20 atomes de carbone ;
ii. de 1 à 19 groupes oxyde d'éthylène ;
b. au moins 50 % de phase hydrophobe en poids de l'agent hydratant solide érodable.

2. Dispositif d'épilation selon la revendication 1, dans lequel l'agent hydratant solide érodable comprend de 1 % à 12 %, de préférence 3 % à 11 %, plus préférablement de 4 % à 10 % d'agent tensioactif modifiant en poids de l'agent hydratant érodable.

3. Dispositif d'épilation selon une quelconque revendication précédente, dans lequel l'agent tensioactif modifiant comprend deux groupes oxyde d'éthylène.

4. Dispositif d'épilation selon une quelconque revendication précédente, dans lequel l'agent hydratant solide érodable a une dureté Chatillon, mesurée selon le « test de dureté Chatillon » décrit ici de 0,50 kg à 3,50 kg, de préférence 0,75 kg à 3,00 kg, plus préférablement 1,00 kg à 2,50 kg.

5. Dispositif d'épilation selon une quelconque revendication précédente, dans lequel la phase hydrophobe comprend une cire et, de préférence, l'agent hydratant érodable comprend de 2 % à 20 % et plus préférablement de 3 % à 15 % de cire en poids de l'agent hydratant solide érodable.

6. Dispositif d'épilation selon une quelconque revendication précédente, dans lequel l'agent hydratant solide érodable comprend un polymère structurant insoluble dans l'eau.

7. Dispositif d'épilation selon la revendication 6, dans lequel le polymère structurant insoluble dans l'eau est soluble dans ou miscible avec la phase hydrophobe au point de fusion du polymère structurant insoluble dans l'eau.

8. Dispositif d'épilation selon la revendication 6 ou 7, dans lequel l'agent hydratant solide érodable comprend de 2 % à 50 %, de préférence de 3 % à 40 %, plus préférablement 4 % à 12 % de polymère structurant insoluble dans l'eau en poids de l'agent hydratant solide érodable.

9. Dispositif d'épilation selon l'une quelconque des revendications 7 à 8, dans lequel le polymère structurant insoluble dans l'eau comprend un copolymère séquencé.

10. Dispositif d'épilation selon une quelconque revendication précédente, dans lequel l'agent hydratant solide érodable comprend de 60 % à 95 %, de préférence de 70 % à 90 % de phase hydrophobe en poids de l'agent hydratant solide érodable.

11. Dispositif d'épilation selon une quelconque revendication précédente, configuré en tant que rasoir comprenant une ou plusieurs lames de coupe.

12. Rasoir selon la revendication 11, dans lequel l'agent hydratant solide érodable est configuré en tant que bande disposée sur l'un et/ou l'autre des côtés de la ou des lame(s) de coupe.

13. Utilisation d'un dispositif d'épilation selon l'une quelconque des revendications précédentes pour éliminer les poils et hydrater la peau.
